(19) Europäisches Patentamt
European Patent Office
Office européen des brevets

(11) Veröffentlichungsnummer: **0 523 508 B1**

(12) **EUROPÄISCHE PATENTSCHRIFT**

(45) Veröffentlichungstag der Patentschrift: **13.09.95**

(51) Int. Cl.[6]: **C07C 68/00**, C07C 69/96

(21) Anmeldenummer: **92111450.0**

(22) Anmeldetag: **06.07.92**

(54) **Verfahren zur Herstellung von Dialkylcarbonaten.**

(30) Priorität: **17.07.91 DE 4123603**

(43) Veröffentlichungstag der Anmeldung:
**20.01.93 Patentblatt 93/03**

(45) Bekanntmachung des Hinweises auf die Patenterteilung:
**13.09.95 Patentblatt 95/37**

(84) Benannte Vertragsstaaten:
**BE CH DE ES FR GB IT LI NL**

(56) Entgegenhaltungen:
**EP-A- 0 425 197**

(73) Patentinhaber: **BAYER AG**
**D-51368 Leverkusen (DE)**

(72) Erfinder: **Landscheidt, Heinz, Dr.**
**Liliencronstrasse 6**
**W-4100 Duisburg 1 (DE)**
Erfinder: **Klausener, Alexander, Dr.**
**Weissdornweg 37**
**W-5190 Stolberg (DE)**
Erfinder: **Wolters, Erich, Dr.**
**Stenzelbergstrasse 11**
**W-5000 Köln 41 (DE)**
Erfinder: **Blank, Heinz Ulrich, Dr.**
**Am Geusfelde 35**
**W-5068 Odenthal (DE)**
Erfinder: **Birkenstock, Udo, Dr.**
**Schneppersdelle 2**
**W-4030 Ratingen 8 (DE)**

EP 0 523 508 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Dialkylcarbonaten durch Umsetzung von Kohlenmonoxid (CO) mit Alkylnitriten in Gegenwart eines Platinmetallhalogenid-Katalysators auf Aluminiumoxiden, Aluminiumoxidhydraten oder Aluminiumhydroxiden als Träger, der Zusätze von Verbindungen weiterer Elemente enthalten kann. Die genannten Träger sind durch eine BET-Oberfläche gekennzeichnet, die größer ist als 1 $m^2/g$.

Dialkylcarbonate sind von allgemeiner chemischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs- und Acylierungsreagenzien. Schließlich haben sie große Bedeutung bei der Herstellung von anderen Carbonaten, von Urethanen und von Harnstoffen.

Es ist bekannt, Dialkylcarbonate durch Umsetzungen von Phosgen bzw. von Chlorameisensäurealkylestern mit Alkoholen herzustellen. Es besteht jedoch ein steigendes Interesse daran, den Einsatz des giftigen Phosgens bzw. der davon abgeleiteten Zwischenprodukte, wie der Chlorameisensäureester, durch andere Verfahren abzulösen. Neben Versuchen, Dialkylcarbonate durch Umsetzung von CO mit niederen Alkoholen zu erhalten, sind insbesondere Verfahren wichtig, in denen CO in der Gasphase mit Alkylnitrit an einem Platinmetall-Katalysator umgesetzt wird. Bei solchen Umsetzungen wird neben dem gewünschten Dialkylcarbonat stets das Auftreten von Dialkyloxalat beobachtet. So ist aus EP 425 197 ein Verfahren bekannt, das gemäß seiner bevorzugten Ausführungsform zu Dialkylcarbonaten des Methanols bzw. Ethanols aus CO und Methyl- bzw. Ethylnitrit in der Gasphase an einem $PdCl_2$-Katalysator auf Aktivkohle führt. Die Selektivitäten zu den gewünschten niederen Dialkylcarbonaten erreichen gemäß dieser EP 425 197, Tabelle 1, Werte bis zu 94 %; jedoch werden als Nebenprodukte stets niedrige Dialkyloxalate und $CO_2$ beobachtet. Bei einer Nacharbeitung konnten darüber hinaus die angegebenen hohen Selektivitäten nur ungenügend reproduziert werden. Die Katalysatoren dieser EP 425 197 enthalten Zusätze von Chloriden unedler Metalle; ein beträchtlicher Zusatz von Chlorwasserstoff, nämlich eine Menge von 1 bis 50 mol-%, bezogen auf das Platinmetall im Katalysator, wird dem System zugesetzt oder ein Teil des Katalysators muß aus dem Reaktor entnommen werden und einer Behandlung mit Chlorwasserstoff unterzogen werden.

Auch in der Zeitschrift für Katalytische Forschung (China), Vol. 10 (1), S. 75-78 (1989), wird als Träger für einen Pd-Katalysator ein Träger aus Kohle eingesetzt, um aus CO und Methylnitrit Dimethylcarbonat zu erhalten, wobei jedoch stets nebenher zusätzlich das Dimethyloxalat entsteht.

Ein Pd/Kohle-Katalysator wird auch in Chin. Sci. Bull. 34 (1989), 875-76 für die Herstellung von Dimethylcarbonat aus CO und Methylnitrit erwähnt.

Diese Bevorzugung eines Kohleträgers ist nicht unerwartet, da in Platinum Metals Review 34 (1990), 178-180 unter Bezugnahme auf ältere Literatur davon berichtet wird, daß bei der Umsetzung eines niederen Alkylnitrits mit CO an einem Pd-Katalysator je nach dem Träger verschiedene Hauptprodukte erhalten werden; ein Kohle-Träger ergibt demnach überwiegend die Dialkylcarbonate, während ein $Al_2O_3$-Träger in der Hauptsache Dialkyloxalate ergibt.

Es wurde nun gefunden, daß bei Verwendung von Aluminiumoxiden, Aluminiumoxidhydraten oder Aluminiumhydroxiden, die eine BET-Oberfläche von mehr als 1 $m^2/g$ besitzen, als Trägermaterialien für Platinmetallhalogenid-Kalalysatoren nicht nur in unerwarteter Weise Dialkylcarbonate erhalten werden, wenn man CO mit Alkylnitriten umsetzt, sondern es wurde darüber hinaus auch noch eine bedeutende Steigerung der Selektivität zu den gewünschten Dialkylcarbonaten erzielt, die soweit geht, daß neben mehr als 97 % Selektivität, in vielen Fällen mehr als 99 % Selektivität zu diesen Dialkylcarbonaten und im allgemeinen überhaupt kein Oxalat nachgewiesen werden kann. Lediglich eine geringe Menge an $CO_2$ kann im Reaktionsgemisch beobachtet werden. Darüberhinaus hat die für nicht möglich gehaltene Verwendung der genannten Träger den wesentlichen Vorteil einer erhöhten Stabilität und Abriebfestigkeit, verglichen mit einem Trägerkatalysator auf der Basis eines Kohleträgers.

Es wurde ein Verfahren zur Herstellung von Dialkylcarbonaten der Formel

$O=C(OR)_2$ (I),

worin

R für geradkettiges oder verzweigtes $C_1$-$C_4$-Alkyl steht,

durch Umsetzung von Kohlenmonoxid (CO) mit Alkylnitriten der Formel

RONO (II),

worin

R die angegebene Bedeutung hat,

in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion gefunden, das dadurch gekennzeichnet ist, daß als Träger Aluminiumoxide, Aluminiumoxidhydrate oder Aluminiumhydroxide mit BET-Oberflächen von mehr als 1 $m^2/g$ eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetall-Verbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1 und einer Temperatur von 50-150 °C gearbeitet wird, wobei absatzweise oder kontinuierlich Halogenwasserstoff mindestens in einer Menge nachgesetzt wird, die geeignet ist, den mit dem Reaktionsgemisch aus dem Reaktor ausgetragenen Halogenwasserstoff zu ersetzen.

Die Reaktion im erfindungsgemäßen Verfahren erfolgt im Sinne der nachfolgenden Reaktionsgleichung:

$$CO + 2\ RONO \rightarrow O{:}C(OR)_2 + 2\ NO.$$

Geradkettiges oder verzweigtes Alkyl mit 1-4 C-Atomen ist beispielsweise Methyl, Ethyl, Propyl, Isopropyl, Butyl, Isobutyl, in bevorzugter Weise die genannten n-Alkyle, in besonders bevorzugter Weise Methyl und Ethyl und in ganz besonders bevorzugter Weise Methyl.

Grundsätzlich ist es möglich, von einem Gemisch verschiedener Alkylnitrite auszugehen, wobei jedoch auch ein Gemisch verschiedener Dialkylcarbonate und gegebenenfalls unsymmetrisch substituierte Dialkylcarbonate entstehen. Im Sinne einer einheitlichen Reaktion ist es daher bevorzugt, von nur einem Alkylnitrit auszugehen.

Während es grundsätzlich möglich ist, CO mit einem Alkylnitrit ohne weitere Gemischkomponente umzusetzen, beispielsweise wenn man sich in der Gemischzusammensetzung außerhalb der Explosionsgrenzen befindet, wird vielfach ein Inertgas zur Verdünnung der Reaktionspartner herangezogen. Inertgase sind beispielsweise Edelgase, Stickstoff und Kohlendioxid, bevorzugt Argon, Stickstoff oder Kohlendioxid, besonders bevorzugt Stickstoff und Kohlendioxid. Die Menge des Inertgases beträgt 20-80 Vol.-%, bevorzugt 30-70 Vol.-%, bezogen auf das gesamte in den Reaktor einzufübrende Gasvolumen. Das Inertgas und gegebenenfalls darin enthaltene nicht umgesetzte Restmengen der Reaktionspartner können recyclisiert werden.

Das Volumenverhältnis der Reaktionspartner Nitrit und CO unter sich beträgt 0,1-10:1, bevorzugt 0,2-4:1, besonders bevorzugt 0,3-3:1.

Das umzusetzende Gasgemisch kann weiterhin geringe Mengen an Alkohol ROH, beispielsweise in einer Menge von 0-10 Vol.-% und geringe Mengen an NO, beispielsweise in einer Menge von 0-10 Vol.-%, beides bezogen auf das Gesamtvolumen des einzusetzenden Gasgemisches, enthalten. Solche Zusätze an ROH bzw. NO können etwa aus der Herstellung des Alkylnitrits stammen und beispielsweise mit diesem in das Reaktionsgasgemisch hereingebracht werden.

Der Katalysatorträger für das erfindungsgemäße Verfahren besteht aus Aluminiumoxid, Aluminiumoxidhydrat oder Aluminiumhydroxid oder einem Gemisch mehrerer von ihnen oder er enthält diese Stoffe. Der Gehalt an diesen Stoffen beträgt 1-100 Gew.-% des gesamten Trägers. Weile unter 100 Gew.-% beziehen sich auf handelsübliche natürliche oder herstellungsbedingte Verunreinigungen, die teilweise nur in Spuren vorhanden sind, beispielsweise $SiO_2$, $Fe_2O_3$, $TiO_2$ CaO, MgO, $Na_2O$, $K_2O$, $B_2O_3$ oder mehrere von ihnen, sowie anorganische Sulfate, Carbonate und/oder Chloride. Die Gehalte an den gesamten Al-oxiden, -oxidhydraten bzw. -hydroxiden betragen hierbei bevorzugt 80-100 Gew.-%, besonders bevorzugt 90-100 Gew.-%. Weitere Werte unter 100 Gew.-% beziehen sich auf Trägerpräparationen, bei denen die genannten Al-Verbindungen auf andere Träger, wie $SiO_2$, $TiO_2$, Bims, $CaCO_3$, $BaSO_4$ oder andere aufgetragen werden und diese anderen Träger schalenförmig umgeben. Die Gehalte an den genannten Al-Verbindungen betragen in diesem letzteren Fall 1-60 Gew.-% der Trägerpräparation, bevorzugt 10-30 Gew.-%.

Die BET-Oberfläche der genannten Al-Verbindungen ist größer als 1 $m^2/g$, bevorzugt 10 $m^2/g$ oder größer, besonders bevorzugt 50 $m^2/g$ oder größer, ganz besonders bevorzugt 100 $m^2/g$ oder größer. Der obere Wert der BET-Oberfläche kann bis zu 500 $m^2/g$, bevorzugt bis zu 400 $m^2/g$ reichen.

Die Al-oxide, -oxidhydrate und -hydroxide werden durch die allgemeine Formel

$$Al_2O_{3-n}(OH)_{2n} \bullet mH_2O$$

beschrieben, in der

n eine der Zahlen 0, 1, 2 oder 3 bedeutet und

m einen Wert von 0 bis 10, bevorzugt 0 bis 5, annimmt.

Der Ausdruck $mH_2O$ steht für entfernbare Wasserphase, die vielfach nicht zum Aufbau des Kristallgitters beiträgt, sodaß m auch Werte nicht ganzer Zahlen annehmen kann. Beim Kalzinieren solcher Trägermaterialien kann m den Wert Null erreichen.

Eine wichtige Al-Verbindung hierunter ist $\gamma$-$Al_2O3$.

Viele der beschriebenen Trägermaterialien sind handelsüblich; Beispiele sind: SPH-501, -508, -517, -535 und -537 von Rhône-Poulenc; CS-331/1, -331/3 und -331/5 von Südchemie; GS-2261/1, -2261/2 und -2261/3 von Kalichemie; Pural-S/SB30, -S/SB50, -S/SB70 und TKA-168 von Condea; D10-10 von BASF; A980 von Hoechst CeramTec; L22 von Girdler.

Die Reaktivkomponente des Katalysators für das erfindungsgemäße Verfahren besteht im reaktionsbereiten Zustand aus dem Platinmetall-Halogenid oder der Platinmetall-Halogenid enthaltenden Komplexverbindung. Solche Komplexverbindungen sind grundsätzlich bekannt und sind beispielsweise Alkalimetallchlorid-Komplexverbindungen, wie Lithium- oder Natrium-tetrachloropalladat, $Li_2[PdCl_4]$ bzw. $Na_2[PdCl_4]$.

Es hat sich weiterhin gezeigt, daß das Platinmetall-Halogenid bzw. die Platinmetall-Halogenid enthaltende Komplexverbindung auch in situ im Reaktor aus metallischem Platinmetall oder einer halogenfreien Platinmetall-Verbindung unter den Reaktionsbedingungen, d.h. in Gegenwart des umzusetzenden Gasgemisches, mit Hilfe von Halogenwasserstoff gebildet werden kann. Der Reaktor kann demnach auch mit einem ansonsten vergleichbaren Katalysator gefüllt werden, der das Platinmetall zunächst in metallischer Form enthält oder der mit Hilfe einer halogenfreien Platinmetall-Verbindung hergestellt worden war. Solche halogenfreien Platinmetall-Verbindungen, die hierfür in Frage kommen, sind beispielsweise Platinmetall-acetate, -nitrate, -propionate, -butyrate, -carbonate, -oxide, -hydroxide oder andere dem Fachmann geläufige. Elemente der Gruppe der Platinmetalle im Sinne der Erfindung sind Pd, Pt, Ir, Ru und Rh, bevorzugt Pd, Ru und Rh, besonders bevorzugt Pd.

Halogenide im Sinne der Erfindung sind Fluorid, Chlorid, Bromid und Iodid, bevorzugt Chlorid und Bromid, besonders bevorzugt Chlorid.

Die Menge des Platinmetall-Halogenids oder der Platinmetall-Halogenid enthaltenden Komplexverbindung beträgt 0,01-8 Gew.-%, bevorzugt 0,05-4 Gew.-%, gerechnet als Platinmetall und bezogen auf das Gesamtgewicht des Katalysators.

Der Katalysator für das erfindungsgemäße Verfahren ist weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet; in einer bevorzugten Weise liegt ein solcher Zusatz vor. Solche Zusätze liegen in salzartiger oder in metallischer Form der genannten Elemente vor. In ähnlicher Weise, wie dies für das Platinmetall weiter oben beschrieben wurde, bildet sich aus der metallischen Form dieser Zusätze und Halogenwasserstoff unter den Reaktionsbedingungen beispielsweise die Halogenidform solcher Zusätze. In bevorzugter Weise bestehen solche Zusätze aus einer Verbindung von Antimon, Wismut, Aluminium Vanadium, Niob, Tantal oder einem Gemisch mehrerer von ihnen, in besonders bevorzugter Weise aus einem Halogenid dieser Elemente, in ganz besonders bevorzugter Weise aus einem Chlorid dieser Elemente. Die Menge des Zusatzes beträgt das 0,1-100fache, bevorzugt das 0,2-10fache der Menge an Platinmetall, gerechnet als Metall sowohl für den Zusatz als auch für das Platinmetall.

Die Herstellung eines erfindungsgemäß einzusetzenden Katalysators erfolgt nach Methoden, die dem Fachmann grundsätzlich bekannt sind. So kann der Träger mit einer Lösung einer der genannten Platinmetall-Verbindungen getränkt oder besprüht werden. In gleicher Weise wird mit dem oder den genannten Zusätzen verfahren. Für den Fall, daß das Platinmetall als Metall oder in Form des Carbonats, Oxids oder Hydroxids auf dem Träger fixiert werden soll und erst im Reaktor in der beschriebenen Weise mit Hilfe von Halogenwasserstoff unter den Reaktionsbedingungen zum Platinmetall-Halogenid aktiviert werden soll, kann die aufgetragene Platinmetall-Verbindung in einer dem Fachmann bekannten Weise durch ein geeignetes Reduktionsmittel zum Metall reduziert werden oder durch ein geeignetes Fällungsmittel in das Carbonat, Oxid oder Hydroxid umgewandelt werden.

Beispiele für die Vielzahl von Präparationen für erfindungsgemäß einsetzbare Katalysatoren und Katalysatorträger sind:

1. Herstellung von Formkörpern aus den genannten oxidischen und hydroxioxidischen Al-Verbindungen.

2. Einarbeitung der katalytisch aktiven Komponenten, wie der oben genannten Platinmetalle bzw. ihrer Verbindungen sowie gegebenenfalls der genannten Zusätze in die Grundmasse für die Träger vor der Formgebung.

3. Nachträgliche Beladung der zuvor hergestellten Formkörper durch Tränken, Tauchen oder Sprühen unter Verwendung von Salzen der Platinmetalle und gegebenenfalls der Zusatzelemente.

4. Aufrollen oder Aufsprühen oxidischer oder hydroxioxidischer Al-Verbindungen auf stückige Massen beliebiger Zusammensetzung und Herkunft.

5. Aufrollen oder Aufsprühen oxidischer oder hydroxioxidischer Al-Verbindungen, die bereits Platinmetalle bzw. ihre Verbindungen sowie gegebenenfalls die genannten Zusätze gemaß 2. eingearbeitet enthalten, auf stückige Massen gemaß 4.

6. Nachträgliche Beladung der gemäß 4. hergestellten Träger gemäß 3.

Es wurde ferner beobachtet, daß es zur Erzielung gleichmäßig hoher Selektivitäten und Raum-Zeit-Ausbeuten an Dialkylcarbonat vorteilhaft ist, Halogenwasserstoff an den Katalysator im Verlauf seiner Einsatzzeit heranzubringen. Es wurde jedoch weiter beobachtet, daß diese Menge an Halogenwasserstoff wesentlich kleiner sein kann, als sie in der Literatur, wie oben angegeben, beschrieben wird. So ist es lediglich erforderlich, die Menge des mit den Reaktionsprodukten ausgetragenen, der aktivierten Form des Katalysator entstammenden Halogenwasserstoffs zu ersetzen. Diese Menge kann analytisch bestimmt werden. Sie erfordert im allgemeinen 50-5 000 ppm Halogenwasserstoff im Reaktandgasgemisch.

Halogenwasserstoff im Sinne der Erfindung ist Fluorwasserstoff, Chlorwasserstoff, Bromwasserstoff und Iodwasserstoff, bevorzugt Chlorwasserstoff und Bromwasserstoff, besonder bevorzugt Chlorwasserstoff.

Der Halogenwasserstoff kann als solcher gasförmig dem Reaktionsgemisch zudosiert werden. Er kann jedoch auch in gelöster Form in einem der im Reaktionsgemisch vorhandenen Stoffe eindosiert werden, so beispielsweise gelöst in dem dem Alkylnitrit zugrunde liegenden Alkohol.

Katalysatoren der beschriebenen Art haben hohe Standzeiten (> 200 h). Über die bereits beschriebene mechanische Stabilität und Abriebfestigkeit hinaus behalten sie ihre Aktivität und Selektivität außerordentlich lange.

Die genannten Katalysatoren können mit 700-17 000 l Gemisch der gasförmigen Reaktionspartner pro l Katalysator und pro Stunde belastet werden (GHSV).

Das erfindungsgemäße Verfahren wird bei einer Temperatur von 50-150 °C, bevorzugt 70-120 °C, besonders bevorzugt 70-110 °C, und einem Druck von 0,8-10 bar, bevorzugt 1-7 bar, besonders bevorzugt 1-6 bar, durchgeführt.

Die Herstellung der erfindungsgemäß einzusetzenden Alkylnitrite erfolgt nach bekannten Verfahren, beispielsweise aus dem zugehörigen Alkohol und salpetriger Säure, die etwa in situ aus einem Alkalinitrit und einer Mineralsäure, wie Schwefelsäure, gebildet wird. Das im Verlaufe des erfindungsgemäßen Verfahrens gebildete Stickstofftnonoxid NO kann kontinuierlich mit Sauerstoff und neuem Alkohol zu Alkylnitrit regeneriert werden (DE-OS 38 34 065) und gemeinsam mit nicht umgesetzten Reaktionspartnern recyclisiert werden.

## Beispiele

Definitionen

Die Raumzeitausbeute (Space Time Yield STY) in [g/l•h] und für Dimethylcarbonat in den Beispielen berechnet sich nach

$$\frac{m_{Dmc}}{v_{Kat} \cdot t},$$

wobei $m_{DmC}$ die Menge gebildetes Dimethylcarbonat (DMC), $\nu_{Kat}$ das Katalysatorvolumen und t die Zeit bedeuten.

Die Selektivität S (%) für Dimethylcarbonat wird berechnet nach

$$S = \frac{n_{DMC}}{n_{DMC} + 2xn_{ODME} + n_{AME} + n_{FDA}} \times 100$$

wobei

| | | |
|---|---|---|
| $^{n}DMC$ | = | Stoffmenge Dimethylcarbonat |
| $^{n}ODME$ | = | Stoffmenge Oxalsäuredimethylester |
| $^{n}AME$ | = | Stoffmenge Ameisensäuremethylester |
| $^{n}FDA$ | = | Stoffmenge Formaldehyddimethylacetal |

bedeuten.

Vergleichsbeispiel 1 (vgl. EP 425 197)

100 ml Aktivkohle-Granulat wurden in bekannter Weise mit einer Lösung von $PdCl_2$ und $CuCl_2$ in Wasser getränkt, das Produkt bei 80 °C im Vakuum (20 Torr) getrocknet. Der fertige Katalysator enthielt 8 g Pd/l und 8 g Cu/l.

Beispiel 1

100 ml $Al_2O_3$-Körper mit einer BET-Oberfläche von 185 $m^2/g$ wurden mit einer wäßrigen $Li_2PdCl_4$ getränkt und das Produkt bei 80 °C im Vakuum (29 Torr) getrocknet. Der Katalysator enthielt dann 8 g Pd/l.

Verfahrensbeschreibung

In einem vertikal aufgestellten Glasrohr wurde zwischen eine Füllung aus Raschig-Ringen der jeweilige Katalysator gebracht.

Das Glasrohr wurde auf 90 °C erhitzt und mit einem Gasgemisch aus HCl, $CO_2$, $CH_3ONO$, CO und $CH_3OH$ beschickt Das dem Reaktor entströmende Gas wurde auf 5 °C gekühlt und die erhaltene kondensierte Phase mittels Gaschromatographie untersucht. Die nicht kondensierten Produkte wurden mittels IR-Spektroskopie und Massenspektroskopie erfaßt.

Tabelle: Beispiele 2 und 3; Vergleichsbeispiele 2 und 3 (gemäß Verfahrensbeschreibung)

| Beispiel | Katalysator | Reaktandengasgemisch (Vol.-%) | | | | | GHSV* | STY | S | STY | S |
|---|---|---|---|---|---|---|---|---|---|---|---|
| | | CO | $CH_3ONO$ | $CH_3OH$ | $CO_2$ | HCl(ppm) | ($h^{-1}$) | ** | ** | *** | *** |
| Vergleichsb. 2 | aus Vergleichsb.1 | 22,5 | 22,5 | 5 | 50 | 1100 | 800 | 120 | 97,0 | 100 | 95,0 |
| Beispiel 2 | aus Beispiel 1 | 22,5 | 22,5 | 5 | 50 | 1100 | 800 | 330 | 99,8 | 330 | 99,8 |
| Vergleichsb. 3 | aus Vergleichsb.1 | 25 | 26 | 6,5 | 42,5 | 1100 | 2400 | 480 | 97,0 | 370 | 94,0 |
| Beispiel 3 | aus Beispiel 1 | 25 | 26 | 6,5 | 42,5 | 1100 | 2400 | 1030 | 99,8 | 1020 | 99,8 |

* GHSV = Gaseous Hourly Space Velocity
** nach 1/2 Stunde
*** nach 10 Stunden

## Patentansprüche

**1.** Verfahren zur Herstellung von Dialkylcarbonaten der Formel

$$O=C(OR)_2,$$

worin

R für geradkettiges oder verzweigtes $C_1$-$C_4$-Alkyl steht,

durch Umsetzung von Kohlenmonoxid (CO) mit Alkylnitriten der Formel

$$RONO,$$

worin

R die angegebene Bedeutung hat,

in Anwesenheit oder Abwesenheit eines Inertgases sowie in Anwesenheit oder Abwesenheit des zugrundeliegenden Alkohols ROH sowie in Anwesenheit oder Abwesenheit von NO an einem Platinmetall-Träger-Katalysator bei erhöhter Temperatur in kontinuierlicher Gasphasenreaktion gefunden, dadurch gekennzeichnet ist, daß als Träger Aluminiumoxide, Aluminiumoxidhydrate oder Aluminiumhydroxide mit BET-Oberflächen von mehr als 1 $m^2/g$ eingesetzt werden, das Platinmetall in Form eines Halogenids oder einer Halogenid enthaltenden Komplexverbindung vorliegt, wobei das Platinmetallhalogenid oder der das Platinmetall enthaltende halogenhaltige Komplex in situ im Verfahrensreaktor aus dem Platinmetall oder einer halogenfreien Platinmetall-Verbindung mit Hilfe von Halogenwasserstoff unter den Reaktionsbedingungen gebildet werden kann, der Katalysator weiterhin mit oder ohne einen Zusatz aus einer Verbindung von Antimon, Wismut, Aluminium, Kupfer, Vanadium, Niob, Tantal, Zinn, Eisen, Kobalt, Nickel oder einem Gemisch mehrerer von ihnen ausgerüstet ist und bei einem Volumenverhältnis von Nitrit:CO = 0,1-10:1, bevorzugt 0,2-4:1, besonders bevorzugt 0,3-3:1, und einer Temperatur von 50-150 °C, bevorzugt 70-120C °, besonders bevorzugt 70-110 °C, gearbeitet wird, wobei absatzweise oder kontinuierlich Halogenwasserstoff mindestens in einer Menge nachgesetzt wird, die geeignet ist, den mit dem Reaktionsgemisch aus dem Reaktor ausgetragenen Halogenwasserstoff zu ersetzen.

2. Verfahren nach Anspruch 1, dadurch gekennzeichnet, daß der Katalysatorträger zu 1-100 Gew.-% des gesamten Trägers aus oxidischen oder hydroxioxidischen Al-Verbindungen besteht.

3. Verfahren nach Anspruch 1, dadurch gekennzeichnet, daß die BET-Oberfläche 10 $m^2/g$ oder größer, bevorzugt 50 $m^2/g$ oder größer, besonders bevorzugt 100 $m^2/g$ oder größer ist.

4. Verfahren nach Anspruch 1, dadurch gekennzeichnet, daß das Platinmetall eines oder mehrere aus der Gruppe von Pd, Pt, Ir, Ru und Rh, bevorzugt eines oder mehrere aus der Gruppe von Pd, Ru und Rh, besonders bevorzugt Pd ist.

5. Verfahren nach Anspruch 1, dadurch gekennzeichnet, daß das Halogen in den Halogeniden und im Halogenwasserstoff Fluor, Chlor, Brom oder Iod, bevorzugt Chlor oder Brom, besonders bevorzugt Chlor ist.

6. Verfahren nach Anspruch 1, dadurch gekennzeichnet, daß in Anwesenheit eines Inertgases gearbeitet wird, wobei der Inertgasanteil 20-80 Vol.-%, bevorzugt 30-70 Vol.-%, des gesamten Gasvolumens beträgt.

7. Verfahren nach Anspruch 1, dadurch gekennzeichnet, daß bei einer Katalysatorbelastung von 700-17 000 l Gemisch der gasförmigen Reaktionspartner pro Stunde und pro l Katalysator gearbeitet wird.

8. Verfahren nach Anspruch 1, dadurch gekennzeichnet, daß Dimethyl- oder Diethylcarbonat durch Umsetzung von CO mit Methyl- oder Ethylnitrit, bevorzugt Dimethylcarbonat durch Umsetzung von CO mit Methylnitrit hergestellt wird.

9. Verfahren nach Anspruch 1, dadurch gekennzeichnet, daß bei einem Druck von 0,8-10 bar, bevorzugt 1-7 bar, besonders bevorzugt 1-5 bar, gearbeitet wird.

10. Verfahren nach Anspruch 1 dadurch gekennzeichnet, daß 50-5 000 ppm Halogenwasserstoff im Reaktandgasgemisch eingesetzt werden.

## Claims

1. Process for preparing dialkyl carbonates of the formula

$$O=C(OR)_2$$

in which

R represents straight-chain or branched $C_1$-$C_4$-alkyl

by reacting carbon monoxide (CO) with alkyl nitrites of the formula

RONO,

in which
R has the meaning given
in the presence or absence of an inert gas and in the presence or absence of the underlying alcohol ROH and in the presence or absence of NO over a supported platinum metal catalyst at elevated temperature in a continuous gas phase reaction, characterized in that aluminas, hydrated aluminas or aluminium hydroxides having BET surface areas of more than 1 $m^2/g$ are used as the support, the platinum metal is in the form of a halide or a halide-containing complex compound, it being possible for the platinum metal halide or the halogen-containing platinum metal complex to be formed in situ in the process reactor from the platinum metal or a halogen-free platinum metal compound by means of hydrogen halide under the reaction conditions, the catalyst is furthermore provided with or without an additive comprising a compound of antimony, bismuth, aluminium, copper, vanadium, niobium, tantalum, tin, iron, cobalt, nickel or a mixture of a plurality thereof, and the reaction is carried out at a nitrite/CO volume ratio of 0.1-10:1, preferably 0.2-4:1, particularly preferably 0.3-3:1, at a temperature of 50-150 °C, preferably 70-120 °C, particularly preferably 70-110 °C, additional hydrogen halide being added batchwise or continuously at least in an amount suitable for replacing the hydrogen halide discharged from the reactor together with the reaction mixture.

**2.** Process according to Claim 1, characterized in that 1-100 °C by weight of the catalyst support, relative to the overall support, consists of aluminium compounds of the oxide or hydroxide type.

**3.** Process according to Claim 1, characterized in that the BET surface area is 10 $m^2/g$ or larger, preferably 50 $m^2/g$ or larger, particularly preferably 100 $m^2/g$ or larger.

**4.** Process according to Claim 1, characterized in that the platinum metal is one or more metals from the group consisting of Pd, Pt, Ir, Ru and Rh, preferably one or more metals from the group consisting of Pd, Ru and Rh, particularly preferably Pd.

**5.** Process according to Claim 1, characterized in that the halogen in the halides and in the hydrogen halide is fluorine, chlorine, bromine or iodine, preferably chlorine or bromine, particularly preferably chlorine.

**6.** Process according to Claim 1, characterized in that the reaction is carried out in the presence of an inert gas, the proportion of inert gas being 20-80% by volume, preferably 30-70% by volume, relative to the overall gas volume.

**7.** Process according to Claim 1, characterized in that the reaction is carried out at a space velocity of 700-17,000 l of the mixture of gaseous reactants per hour and per l of catalyst.

**8.** Process according to Claim 1, characterized in that dimethyl carbonate or diethyl carbonate is prepared by reacting CO with methyl nitrite or ethyl nitrite, preferably dimethyl carbonate is prepared by reacting CO with methyl nitrite.

**9.** Process according to Claim 1, characterized in that the reaction is carried out at a pressure of 0.8-10 bar, preferably 1-7 bar, particularly preferably 1-5 bar.

**10.** Process according to Claim 1, characterized in that 50-5,000 ppm of hydrogen halide are used in the gas mixture of reactants.

**Revendications**

**1.** Procédé de préparation de carbonates de dialkyle de formule

$O=C(OR)_2$,

dans laquelle

R représente un groupe alkyle à chaîne droite ou ramifiée en $C_1$-$C_4$,

par réaction de l'oxyde de carbone (CO) avec des nitrites d'alkyle de formule

RONO,

dans laquelle

R a les significations indiquées ci-dessus,

en présence ou non d'un gaz inerte et en présence ou non de l'alcool correspondant ROH et en présence ou non de NO sur un catalyseur consistant en un métal de la série du platine sur support à température élevée dans une réaction continue en phase gazeuse, caractérisé en ce que l'on utilise en tant que supports des alumines, des alumines hydratées ou des hydroxydes d'aluminium ayant des surfaces BET supérieures à 1 $m^2/g$, le métal de la série du platine est à l'état d'halogénure ou de complexe contenant un halogénure, l'halogénure de métal de la série du platine ou le complexe contenant un tel halogénure pouvant être formé in situ dans le réacteur à partir du métal de la série du platine ou d'un dérivé de ce métal exempt d'halogène à l'aide d'un halogénure d'hydrogène dans les conditions de la réaction, le catalyseur pouvant en outre le cas échéant contenir des adjonctions d'un dérivé de l'antimoine, du bismuth, de l'aluminium, du cuivre, du vanadium, du niobium, du tantale, de l'étain, du fer, du cobalt, du nickel ou d'un mélange de plusieurs de ces métaux, et on opère à un rapport en volume nitrite/CO = 0,1 à 10 : 1, de préférence 0,2 à 4 : 1, plus spécialement 0,3 à 3 : 1, à une température de 50 à 150 °C, de préférence de 70 à 120 °C et plus spécialement de 70 à 110 °C, en ajoutant par portions ou en continu des compléments d'halogénure d'hydrogène en quantité au moins suffisante pour remplacer l'halogénure d'hydrogène évacué du réacteur avec le mélange de réaction.

**2.** Procédé selon la revendication 1, caractérisé en ce que le support de catalyseur consiste pour 1 à 100 % de son poids en les oxydes ou hydroxydes d'aluminium mentionnés.

**3.** Procédé selon la revendication 1, caractérisé en ce que la surface BET est de 10 $m^2/g$ ou plus, de préférence de 50 $m^2/g$ ou plus et plus spécialement de 100 $m^2/g$ ou plus.

**4.** Procédé selon la revendication 1, caractérisé en ce que le métal de la série du platine consiste en un ou plusieurs métaux du groupe formé par Pd, Pt, Ir, Ru et Rh, de préférence un ou plusieurs du groupe formé par Pd, Ru et Rh et tout spécialement Pd.

**5.** Procédé selon la revendication 1, caractérisé en ce que l'halogène des halogénures et de l'halogénure d'hydrogène consiste en fluor, chlore, brome ou iode, de préférence chlore ou brome, et tout spécialement chlore.

**6.** Procédé selon la revendication 1, caractérisé en ce que l'on opère en présence d'un gaz inerte dont la proportion est de 20 à 80 %, de préférence de 30 à 70 % du volume total des gaz.

**7.** Procédé selon la revendication 1, caractérisé en ce que l'on opère à une charge du catalyseur représentant de 700 à 17 000 l du mélange des réactifs gazeux par heure et par litre de catalyseur.

**8.** Procédé selon la revendication 1, caractérisé en ce que l'on prépare le carbonate de diméthyle ou le carbonate de diéthyle par réaction de CO avec le nitrite de méthyle ou d'éthyle et de préférence le carbonate de diméthyle par réaction de CO avec le nitrite de méthyle.

**9.** Procédé selon la revendication 1, caractérisé en ce que l'on opère à une pression de 0,8 à 10 bar, de préférence de 1 à 7 bar et plus spécialement de 1 à 5 bar.

**10.** Procédé selon la revendication 1, caractérisé en ce que l'on introduit de 50 à 5 000 ppm d'halogénure d'hydrogène dans le mélange de réaction gazeux.